# EUROPEAN PATENT APPLICATION

(11) **EP 2 801 359 A2**
(43) Date of publication of application: **12.11.2014**
(21) Application number: 14170572.3
(22) Date of filing: 08.05.2006
(51) Int. Cl.: A61K 31/465, A61P 31/00

(54) **The use of nicotine for the treatment of sepsis in humans caused by antibiotic**

(62) Divisional of application: 06747531.9
(71) Applicant: Solis Herrera, Arturo, CEP-20000 Aguascalientes, Ags. (MX)
(72) Inventor: Solis Herrera, Arturo, CEP-20000 Aguascalientes, Ags. (MX)
(74) Representative: Vogel, Andreas

(57) **Abstract**

The invention relates to nicotine, analogues thereof, precursors thereof, or its derivatives in effective dose form and adequate pharmacophore for the treatment of sepsis in humans and/or animals, in particular in mammalians, caused by antibiotic.

## Description

### OBJECTIVE OF THE INVENTION

This invention protects the use of substances that promote, facilitate or intensify the releasing and the action or activity of α-MSH hormone (melanocyte stimulating hormone), as nicotine, analogues thereof, precursors thereof or derivatives through its indirect effect mainly on the melanotrophs located in the pars intermedia of the hypophysis in close relationship with lactotrophs. There are different susceptible pathological conditions the which can be improved by administration of α-MSH, because the stem cells that respond to said stimulus participate in several mainly functions in the organism, among them we have as an example, (but not limitated): proliferatives retinopathies where the eye fibroblasts, as any organism fibroblast, in presence of hypoxia reacts secreting collagen (Dr. Humberto Montoya de Lira, 2000), initial stages of retrolental fybroplasia, the proliferative diabetic retinopathy, the post traumatic proliferative retinopathy; the proliferative retinopathy caused by hypoxia: primary, secondary, local and distant; infectious syndrome where secondary alterations of liver, kidney and lung may be prevented; in conditions where α-MSH is a protective factor against the degenerative osteoarthrosis, against eclampsya, against Parkinson desease, against Alzheimer, against arthritis from different etiology, against the rejection of transplanted tisues, improves depression, diminish in a 95 % the tissue deterioration in experimental models of ischemia/reperfussion in kidney, lung, intestine, protects vessels from deterioration caused by bacterians LPS (lipopolysacharides), protects liver from deterioration induced by LPS, at the same time α-MSH is considered a protective factor in degenerative osteoarthrosis it seems to protect cartilage. Also antidepressive effect has been described for α-MSH, which can have an important therapeutic role in obesity control.

### BACKGROUND OF THE INVETION

The α-MSH is a three decapeptide with a potent anti inflammatory action, with prominent actions reducing the inflammatory mediators for example, reduces the level of tumor necrosis factor, including cytokines. Alpha-MSH hormone is a compound of 13 amino acid derivated from propiomelanocortin, it expresses in several regions of the Central Nervous System and in peripheral cells, including fagocytes, and keratinocytes The anti-inflammatory effects are mainly through the antagonism of proinflammatory mediators including α-Tumor Necrosis Factor, Interleukina 6 and nitric oxide (NO). The α-MSH neuropeptide is an endogenic modulator of inflammation. The idea that α-MSH is important in the host responses begins from the inicial observation from the antipyretic properties of the molecule. The α-MSH potency for reducing the fever as resulted of endogenous pyrogens is dramatic: 20 000 (twenty thousand) times as great as acetaminophen (Airagui, Lorena 2000) when the relation molecule to molecule is compared. Alpha-MSH also inhibits fever caused by endotoxin, IL-6 and alpha-TNF (α-TNF), so it has an inhibitor effect on IL-1, and on the increase induced by α-TNF in the circulating proteins from acute stage and neutrophyles. So α-MSH also inhibits the tissue trauma in systemic inflammation models as acute respiratory syndrome and peritonytis caused by cecal ligation and punction as well as in isquemic acute renal defect.

Mortality, by combination of acute renal insufficiency and acute respiratory insufficiency reaches an 80 %. The severe trauma, burns, haemorrhages, sepsis, shock, or severe local tissular trauma, can iniciate a systemic inflammatory response provoking the multiple organic failure and death. There are pathogenic and epidemiological connection between renal and lung trauma. A great part of risk increase due to acute renal failure after heart surgery comes from extra renal complications such as respiratory failure

Severe tissular trauma happened after a prolongated ischemia in the inferior torso or during a complicated surgery of aorthic abdominal aneurism or during an acute respiratory failure syndrome.

In animal models, secondary pulmonary trauma (or distant) can be started by severe local ischemia in liver, in the gastrointestinal tract, in inferior member, kiney or chemical pancreatytis. For example, renal traumatism by ischemia/reperfussion, can increase lung vascular permeability, as well as produce intersticial edema, alveolar haemorrhage and damage of reological properties of eritrocytes. Due to lung has the biggest microcapilar trauma in the organism, responds to circulating proinflammatory signs with activation of lung macrophages, secretion of proinflammatory cytokines, attraction of neutrophyls and macrophages, finally resulting a lung trauma.

There are many similarities in the activation of local pathways of tissular trauma, after pulmonary trauma and acute renal and secondary pulmonary traumatism. The renal ischemia/reperfussion causes apoptosis and necrosis in rectum proximal tubules and inflammatory infiltration of leukocytes. Earlier in the reperfussion period, there is an activation of activated kinases by stress (for example, kinase protein p-38 activated by mitogens [MAPK]) and by transcription κB factor of nuclear factors (NF-κB) and protein activator (AP-1) and induction of proinflammatory cytokines (α-TNF and adhesion molecules (intercellular adhesion molecule-1 [ICAM-1]). The selective inhibition of α-TNF and/or of ICAM-1 diminishes acute renal trauma. Parallely, proinflamatory traces NF-kB, p-38 and AP-1 are activated after acute pulmonary trauma, and the inhibition of NF-κB and p-38 reducing distant pulmonary trauma or secondary. Although, there is no agent has shown to inhibit both local trauma and distant pulmonary trauma. For example the inhibitor of p-38 CNI-1493 partialy reduces distant pulmonary trauma but does not have effect in subyacent renal trauma by ischemia/reperfussion.

Alpha-MSH hormone (α-stimulant of melanocytes) is an anti inflammatory citokyne that inhibits chronic or acute systemic inflammation. Alpha-MSH inhibits renal trauma by ischemia/reperfussion, by cysplatin administration, or after a transplant by marginal donor; but not after administration of mercury.

### (Mercury poisons melanocytes).

Mechanism of action of α-MSH is extense, and the actions documented by us are: the inhibition of inflammatory traces, cytoxics, and apopotics pathways activated by renal ischemia.

It has been demostrated that α-MSH inhibits activation of α-TNF and ICAM-1 four hours after the reperfussion. Although, the earlier molecular mechanisms activated by α-MSH are not elucidated. In models of ischemia/reperfussion disease and other similars, α-MSH inhibits the production of many cytokines, chemokines, and the inducible synthase of nitric oxide; this suggests that α-MSH acts in one or several early common steps in inicial pathway of inflammation. Recent studies have demonstrated that α-MSH suppresses the stimulation of NF-κB in brain inflammation and in cell culture exposed to LPS (lipopolysacharides).

Alpha-MSH also inhibits p38 MAPK in melanoma cells B16 and in AP-ligand-DNA in dermic fibroblasts, but not in macrophages. By means, it has been determined that α-MSH diminishes pulmonary trauma caused by renal trauma from ischemia/reperfussion.

In animal models, it has been demostrated that serum creatinine increases, in an important form, at 4, 8 and 24 hours after renal ischemia/reperfussion in comparison with witness and control animal. At the same time, animals that received α-MSH had important lower levels of creatinine than animals that only had vehicles as well as less cylinders and necrosis evaluated by quantitative citology at 4 hours.

**Effects of α-MSH in leukocytes accumulation:** Preliminary studies have shown that renal ischemia causes leukocytic infiltration in kidney and lung and α-MSH inhibits locally leukocytes accumulation after acute inflammation and in renal ischemia. The stain with stearate of chloroacetate shows an increase in leukocyte accumulation in lung four hours after renal ischemia/reperfussin compared with witness animals. Treatment with α-MSH before releasing of patch (clamp) diminishes leukocyte infiltration. These changes were evaluated by counting positive stearase cells in lung and kidney. There were elevated infiltrating leukocytes in lung and kidney in very early stages after renal trauma by ischemia/reperfussion, and said accumulation was inhibited by α-MSH

**Effects of α-MSH on α-TNF and ICAM-1.** Renal ischemia increases α-TNF and ICAM-1 and the inhibition of both pathways diminish, in a dramatic form, the renal damage. It has been found that renal ischemia causes phosphorylation (by means, activation) from IκBα cytosolic in kidney as well as in lung during 15-30 minutes after reperfussion. Administration of α-MSH just before releasing of patch (clamp) inhibits phosphorylation of IκBα as in kidney as in lung.

Phosphorylation of IκBα causes its own destruction; it allows that the dimers of NF-κB containing p65 traslocated to the nucleus. As it was to hope, phosphorylation of IκBα lets appear p65 in the nucleus rapidly in kidney as well as in lung, which could be inhibited by administration of α-MSH.

The activity ligand NF-κB increased rapidly in lung as well as in kidney at the end of the ischemia period, the treatment with α-MSH inhibited the ligand NF-κB activity in kidney and lung.

Renal ischemia/reperfussion also causes a rapid phosphorylation (and of course activation) from p38 of kidney and lung without changes in the total p38. Phosphorylation of p38 was inhibited with α-MSH treatment.

It has been found inflammatory cells infiltrating, intensely and rapidly, the lung after renal ischemia; and it has been proved that α-MSH has a dramatic effect in pulmonary trauma, because it inhibites pulmonary infiltration in 4 and 8 h after renal ischemia, with similar effects on kidney. Effect of α-MSH is more dramatic at 8 hours than in 4, may be because can inhibit most early responses of stress/inflamatories, some or all of them can contribute to the ability of α-MSH for diminishing the progress of damage.

It has been found that renal ischemia/reperfussion increases levels of mRNA (messenger) for α-TNF- and ICAM-1 after cysplatin inhibition.

Alpha-TNF is important in pathogenesis of distant organ damage; because antibodies against α-TNF reduce pulmonary damage after liver ischemia and agents that diminish distant pulmonary damage also diminish α-TNF located in pulmonary tissue. This evidence suggests the importance of inflamation and α-TNF particularly in distant pulmonary trauma induced by ischemia or damage to extra pulmonary organs.

Some of α-MSH effects are probably mediated by direct effect on leukocytes, because the neutrophyls and macrophages express receptors for α-MSH.

Alpha-MSH inhibits the migration of neutrophyls in vitro and the production of nitric oxide in culture of macrophages. Although α-MSH inhibits damage by renal ischemia/reperfussion until in absence of leukocytes infiltration, which suggests that α-MSH can also act by different ways from leukocytes.

Administration of α-MSH just before reperfussion has a great protective effect in lung as well as in kidney. Alpha-MSH reduces, in a dramatic form, the activation of distant or secondary pulmonary damage caused by lung transplant, pancreatytis, liver ischemia, haemorrhages or secondary reactions to bacterian lipopolysacharides.

The events that cause distant renal damage after renal ischemia/reperfussion are unknown.

Pretreatment for distant ischemia that inhibits terminal-kinase C-Jun N and activation of p38, prevent renal damage after ischemia/reperfussion, but unfortunately, it is not a viable therapeutic alternative, is much more practical the administration of α-MSH.

There are more evidences every time that suggest the activation of NF-κB proceeds and may cause secretion of α-TNF after myocardial ischemia and kidney ischemia.

The combined acute lung and kidney failure comes with an extremely high morbility and mortality, whose subyacent mechanisms are unknown The severe tissular trauma presented in the burnt, in the polytraumatized patients, in the ischemia prolongated of the inferior torso or complicated surgery by abdominal aneurism aorthic, frecuently provoke the subsequent events that cause the multiple organic failures. Actually therapeutic steps available are very elementals and are limited to replace the function of the lost organ, controlling ventilation and dialysis; preventing barotrauma, and optimizing cardiovascular function with resucitation of the adequate volumen and inotropic support. Treatment with medication is not desirable. Recently, C-protein activated showed some utility to diminish death by sepsis. Additional strategies to prevent and/or treat multiple organic failures will be extremely useful. In this moment we do not know any medicine that reduces pulmonary damage nor renal damage.

It has been demostrated that administration of α-MSH just before reperfussion inhibits acute renal damage as well as pulmonary damage.

The ability of α-MSH to inhibit the damage in both organs, the extension of protection that reaches or gets, and the wide action mechanism distinguishes α-MSH from other agents used to prevent, limit, protect or delay damage by ischemia/ reperfussion.

This suggests that α-MSH can have an important therapeutic effect on adequate patients. (Deng, Hu, Yuen, Star, Am J Respir Crit Care Med Vol 169 pp 749- 756, 2004.).

Alpha-MSH can have an important therapeutic role for treatment of vasculitis, sepsis, chronic and acute inflammatory diseases from different ethiology. (Endocrinology 144: 360-370, 2003).

In intermittent hemodialysis, it can characteristically appreciate elevation of α-TNF, IL-6 and NO; so that α-MSH is liberated in these patients to counteract the proinflammatoriy effects of these cytokines (Lorena Airagui, Leticia Garofalo, Maria Grazia Cutuli. Nephrol Dial Trans 2000 (15):1212-1216).

Alpha-MSH modulates α-TNF local and circulating in experimental models of brain inflammation (Nilum Rajora, Giovanni Boccoli, Dennos Burns.The Journal of Neuroscience March 15, 1997; 17(6): 2181-2186.) In this research, the secretion of α-TNF in central nervous system was induced by a local injection of bacterian LPS. The plasma concentration of α-TNF had an important elevation after central application of LPS, indicating that the host peripheral response was increased by induction of CNS sign.

The inhibition of α-TNF synthesis by α-MSH was confirmed using inhibition of mRNA. Although some inflammatory cytokines contribuye to Central Nervous System inflammation (CNS), α-TNF is specially important because it is identified as an important agent in physiopathogenic of CNS diseases as multiple sclerosis, HIV infection of CNS, Alzheimer disease, meningitis, severe cranium encephalic traumatism consequently to the ischemia/reperfussion and/or trauma. The increase of α-MSH levels, by endogenic or exogenic administration, has an important therapeutic or prophylactic effect for dimishing the diseases with α-TNF increased, as above mentioned.

**Effect of α-MSH in Central Nervous System (CNS) degeneration.** Almost every one of degenerative diseases of CNS is associated with chronic inflammation. An important stage in this process is the activation of brain fagocytic mononuclear cells named microglia. It has been reported the nicotine neuroprotector effects due to its action over selective nicotinic antagonist receptors: α7; in illneseses as Parkinson, Alzheimer, Depression, Obesity, Ageing etc. (Cholinergic modulation of microglial activation of α-7 nicotic receptors. R Douglas Shytle, Takashi Mori, Kira Townsed, Journal of Neurochemistry , 2004, 89, 337-443.)

It is congruent that beneficial effects of α-MSH include whole organism as: skin, mucous, eyes, intestine, muscle, joints, etc, because they have common methabolic pathways stimulated by said hormone.

### DETAILED DESCRIPTION OF THE INVENTION.

The invention mainly consists in administration of nicotine, analogues, thereof precursors, thereof or its derivates to adequate patients, in pharmacophores and effective dosis, by the suitable pathway in each case; in therapeutic form and or prophylactic, through its effect on hypothalamus (main action but not the unique), the α-MSH releasing induced by melanotrophs from pars intermedia of the hypophysis, because this secretion (α-MSH) is tonic. By means the hypothalamus has a suppressor effect more than secretor, to difference to others hypothalamic effect on hypophysis. It seems to be one of the few hypophysary hormone released .in tonic form (constant) and the hypothalamus inhibits this releasing through the dopamine secretion (hypothalamic) (another hypophysary hormone released in tonic form is the prolactine from mamotrophs). The nicotine precursors thereof analogues, or derivates, administrated by adequate form in effective dosis and adequate pharmacophore, provoke an effect on hypothalamus diminishing of dopamine secretion, and the melanotrophs of the pars intermedia of the hypophysis releasing α-MSH tonically, (to more hypothalamic inhibition less tonic α-MSH release and viceverse), as it happens during all its life, as resulted of several factors, said secretion is diminished and/or inclusive interrupted; said factors can be environmental, emocional, differents acute or chronic diseases, infectious diseases, surgeries, different therapeutic actions, pesticides, hormone, chemical agents, different xenobiotics types, etc., inciding the α-MSH secretion in all cases, in one or another sense. The action of nicotine suggested in this patent and maybe not only the unique effect of nicotine, analogues thereof, precursos thereof, or derivates, said action is to provoke the α-MSH releasing mainly from melanotrophs located in the pars intermidia of the hypophysis in close contact with mamotrophs, although it is not the unique pathway, the which maybe documented in complete form, more scientific, because up to now it is the only way in which we can document in the more complete and scientific form, without rulling out other action sites the skin (keratinocytes), pilose follicle, etc

### About the use nicotine in this patent we have some examples

Example 1. Female patient 27 years old she is in the ninth month of pregnant without diabetes or hypertension or neuropathy antecedents. No surgery antecedents begins with a intense pain in the right renal region in 72 hours of evolution, she can not sleep, requires the administration of analgesics every three hours. Tweenty four hours later Amikacina IM every 12 hours was administered. She could not be in a free attitude due the intense pain, apart from the natural upsets in the ninth pregnat month. It was decided to administrate nicotine in watery vehicle by sublingual pathway in a concentration of 3 mg/ml.

At the beginning 15 drops were administrated and 30 minutes later 10 drops more. Patient slept and after three days she could sleep all night, pain diminished significantly that she did not awake. General physical state improves in the dramatic form, the analgesic was limited to a half aspirine every 12 hours and the antibiotics course continued for 8 days more. Nicotine was administrated during 4 weeks in dosis of 5 drops by sublingual pathway every three hours.

Example 2. Male patient just born, (his mother is patient from example 1) born by cesarean who had in the first hours hypothermia and vomit, few hours after appeared petequias in the back, in blood analysis it was found plateletopenia and increase of sedimentation velocity analysis, considering that it was a sepsis amikacina IV was administered initially; agree to mother treatment, it was began the administration of nicotine by sublingual pathway in a dosis of 1 drop every 12 or 24 hours; in concentration of 3 mg/mL. Patient slept deep and long, curiously the heart increased its rate from 110 per minute to 130 and the periferal oxygen was not diminished of 93%. Tweenty four hours later the baby had increased 80 grams weight. Now the kid is growing well and without consequences.

Example 3. Male patient 25 years old with post traumatic bleeding (hyphema). She was reviewed at 14^{th} of its disease, and the hyphema of 90% did not improve with the first treatment, the patient came to us because his doctor suggested him a surgery to evacuate blood for avoiding loose his eye. We explained to patient the treatment to stimulate α-MSH could be an alternative form in order to protect the tissue from apoptosis as a potent anti-inflammatory agent, when the nicotine induces the α-MSH release. We indicated a dosis of 2 drops sublingual pathway every hour, for the hyphema was of 90% the vision was poor and the intraocular preasure was 40 mmHg despite last treatment. All medicine was suspended and began the new treatment. Three weeks later vision was 20/40. The recovery was dramatic and complete in a 90% after four weeks.

## Claims

1. Nicotine, analogues thereof, precursors thereof, or its derivatives in effective dose form and adequate pharmacophore for the treatment of sepsis in humans and/or animals, in particular in mammalians, caused by antibiotic.

2. Nicotine, analogues thereof, precursors thereof, or its derivatives according to claim 1 wherein sepsis in human caused by amikacina is treated.

3. Nicotine, analogues thereof, precursors thereof, or its derivatives according to claim 1 or 2, for the administration by sublingual pathway delivery.

4. Nicotine, analogues thereof, precursors thereof, or its derivatives according to any one of the preceding claims for the delivery in a concentration of 3 mg/ml.

5. Nicotine, analogues thereof, precursors thereof, or its derivatives according to any one of the preceding claims, for the administration in a watery vehicle.

6. Nicotine, analogues thereof, precursors thereof, or its derivatives according to any one of the preceding claims, for administration by sublingual pathway in a dose of 10 drops.

7. Nicotine, analogues thereof, precursors thereof, or its derivatives according to claim 6, which is intended to administer every 12 or 24 hours.

8. Nicotine, analogues thereof, precursors thereof, or its derivatives according to any one of the preceding claims, for delivery in infant humans and/or animals, in particular in infant mammalians.

9. Nicotine, analogues thereof, precursors thereof, or its derivatives according to any of the preceding claims for increasing heart rate while peripheral oxygen is not dimished.

10. Nicotine, analogues thereof, precursors thereof, or its derivatives according to any of the preceding for the modulation of alpha-MSH in cells located in the pars intermidia of the hypophysis.

11. Nicotine, analogues thereof, precursors thereof, or its derivatives according to claim 10 **characterized in that** alpha-MSH release is enduced and/or provoked.

12. Nicotine, analogues thereof, precursors thereof, or its derivatives according to any one of the preceding claims for therapeutic and/or prophylactic purpose in humans and/or animals, in particular in mammalians.
